# EUROPEAN PATENT APPLICATION

(11) **EP 3 882 006 A1**
(43) Date of publication of application: **22.09.2021**
(21) Application number: 19904876.0
(22) Date of filing: 12.12.2019
(51) Int. Cl.: B29C 64/188, B29C 64/147, B29C 64/118, B29C 64/209, B29C 65/02, A61C 8/02, A61F 2/28, A61L 27/14, C08L 67/04, C08L 67/02

(54) **METHOD FOR MANUFACTURING ALVEOLAR BONE REGENERATION MEMBRANE**

(30) Priority: 28.12.2018 KR 20180171560
(71) Applicant: Osstemimplant Co., Ltd., Seoul 08505 (KR)
(72) Inventor: JANG, Ja Yong, Busan 49344 (KR); KWON, Doo Yeon, Busan 47749 (KR); JANG, Il-Seok, Yangsan-si, Gyeongsangnam-do 50653 (KR); SONG, Ju Dong, Busan 48272 (KR)
(74) Representative: Isarpatent
(86) International application number: PCT/KR2019/017576
(87) International publication number: WO 2020/138779

(57) **Abstract**

An embodiment of the present invention provides a method for manufacturing an alveolar bone regeneration membrane, the method comprising: (A) a step for preparing a base film by melting and molding a synthetic polymer resin; (B) a step for pressing the base film to prepare a first polymer film; and (C) a step for alternately laminating and bonding the first polymer film and a second polymer film containing a natural polymer resin. The method satisfies the conditions below. The average pore size of the base film and the first polymer film are D₁ and D₂, respectively, and 0.1 ≤ D₂/D₁ ≤ 0.5.

## Description

### [Technical Field]

The present invention relates to a method for manufacturing an alveolar bone regeneration membrane, and more specifically, to a method for manufacturing an alveolar bone regeneration membrane, which is easy to mold, has excellent space retention ability after molding, and has improved convenience of surgery.

### [Background Art]

There is an increasing number of cases in which the function of teeth is lost due to the aging of the population, the alveolar bone is lost due to long-term neglect of tooth defects, or the function cannot be properly performed. When only the function of teeth is lost, the function can be restored by placing an artificial tooth through an implant surgery, but when the alveolar bone is lost, a new alveolar bone should be formed through a bone graft and then an artificial tooth should be placed.

Specifically, when the bone quality is poor or the amount of bone is insufficient during correction surgery or implant surgery, or when the alveolar bone is destroyed or lost due to periodontitis, a surgery to increase the alveolar bone is required, and when bone regeneration (bone composition) is required as described above, guided bone regeneration (GBR) or bone grafting is performed. The guided bone regeneration is an alveolar bone regeneration technique in which a membrane is placed on a bone regeneration site and a protective barrier for growth of alveolar bone is formed while blocking the invasion of undifferentiated fibroblasts. In the case of guided bone regeneration, a membrane is required to provide an appropriate space to the bone regeneration site.

Typically used membranes include polytetrafluoroethylene (PTFE), collagen, titanium, and the like.

A collagen membrane is generally an absorbent membrane that uses collagen obtained from animals, and has the advantages of a high success rate of bone regeneration and no need for removal surgery, but most of the absorbent membranes have problems in that due to reduced robustness of the membrane itself, it is not easy to fix a bone grafting material to the grafted part and the elasticity may cause poor retention after molding, and the membrane is ruptured or pushed, and thus may be difficult to fix in an accurate position, thereby causing the bone grafting material to fall off or move. In addition, since the absorbent membrane may be absorbed at an early stage, the bone tissue regeneration effect may be reduced.

It is known that a titanium membrane is a non-absorbent membrane, and has excellent space-retaining power derived from the robustness inherent to metal, and thus enables a large amount of bone grafting. However, the titanium membrane requires additional molding depending on the periodontal morphology of an individual patient, and there is an inconvenience that the titanium membrane should be removed through a separate secondary surgery after alveolar bone regeneration. In particular, due to a phenomenon that a bent portion locally protrudes excessively during the molding process, the protrusion may penetrate the gingiva during the bone regeneration induction period to cause infection from the outside.

Furthermore, a PTFE membrane is a non-absorbent membrane, and has advantages in that the PTFE membrane is not harmful to the human body due to excellent biocompatibility, has excellent shielding stability, and retains existing bones and artificial bones in their original shape. However, the PTFE membrane should be removed through a secondary surgery like the titanium membrane, and plaque may be deposited on an exposed site of the membrane, thereby causing infection.

In this regard, Korean Registered Patent No. 10-1649125 provides a method for manufacturing an alveolar bone regeneration membrane in which a biomaterial is laminated on a biodegradable polymer, and realized high space retention ability by the method. However, the alveolar bone regeneration membrane is provided in a thermoformed state with a bent structure, and thus has a limitation in terms of convenience of surgery because it is difficult for a practitioner to mold a membrane according to the oral structure and shape of a patient.

### [Disclosure]

### [Technical Problem]

The present invention has been made to solve the above-described problems in the related art, and an object of the present invention is to provide a method for manufacturing an alveolar bone regeneration membrane, which is easy to mold, has excellent space retention ability after molding, and has improved convenience of surgery.

### [Technical Solution]

An aspect of the present invention provides a method for manufacturing an alveolar bone regeneration membrane, the method comprising: (A) a step of preparing a base film by melting and molding a synthetic polymer resin; (B) a step of pressing the base film to prepare a first polymer film; and (C) a step of alternately laminating and bonding the first polymer film and a second polymer film containing a natural polymer resin. The method satisfies the conditions below. The average pore size of the base film and the first polymer film are D₁ and D₂, respectively, and 0.1 ≤ D₂/D₁ ≤ 0.5.

In an embodiment, the synthetic polymer resin may be one selected from the group consisting of polylactic acid (PLA), polyglycolic acid (PGA), poly lactic-co-glycolic acid (PLGA), poly(L-lactic acid) (PLLA), polycaprolactone (PCL), poly(lactide-co-caprolactone) (PLCL), polyhydroxybutyrate (PHB), polyhydroxyvalerate (PHV), polydioxanone (PDO), poly(trimethylene carbonate) (PTMC), and combinations of two or more thereof.

In an embodiment, in the step (A), the molding may be performed by additive manufacturing.

In an embodiment, the additive manufacturing may be performed using a fused deposition modeling (FDM) type 3D printer including one or more nozzles.

In an embodiment, the nozzle may have a diameter of 0.1 to 0.5 mm.

In an embodiment, in the step (B), the pressurization may be performed at a pressure of 3 to 50 mPa.

In an embodiment, the natural polymer resin may be one selected from the group consisting of collagen, chitosan, hyaluronic acid, carboxymethyl cellulose, heparan sulfate, dextran, alginate, and combinations of two or more thereof.

In an embodiment, the second polymer film may be prepared by a method including the following steps. (b1) a step of dissolving a second polymer resin in an acid solution; (b2) a step of fiberizing the second polymer resin by introducing a salt into the product of the step (b1); (b3) a step of drying the product of the step (b2); (b4) a step of cross-linking the second polymer resin by introducing a cross-linking agent into the product of the step (b3); and (b5) a step of washing and drying the product of the step (b4) and then pressurizing.

In an embodiment, the acid solution may include one selected from the group consisting of phosphoric acid, sulfuric acid, hydrochloric acid, nitric acid, acetic acid, and combinations of two or more thereof.

In an embodiment, the salt may be one selected from the group consisting of a phosphate, a calcium salt, a sodium salt, and combinations of two or more thereof.

In an embodiment, the cross-linking agent may be one selected from the group consisting of formaldehyde, a-hydroxyadipaldehyde, glutaraldehyde, 1-ethyl-3-(3-dimethylaminopropyl)-carbodiimide/N-hydroxy succinimide (EDC/NHS), and combinations of two or more thereof.

In an embodiment, the step (C) may include the following steps. (c1) a step of alternately laminating the first and second polymer films; and (c2) a step of heating the product of the step (c1) to a temperature of 30 to 80°C and bonding the product at a pressure of 3 to 30 mPa.

In an embodiment, in the step (C), the second polymer film including the natural polymer resin may be laminated and bonded on at least one surface of the first polymer film.

### [Advantageous Effects]

According to an aspect of the present invention, during the manufacture of a synthetic polymer film used for a base layer or core layer in an alveolar bone regeneration membrane with a structure in which a plurality of layers is laminated, by adjusting an average pore size depending on pressure to a predetermined range and laminating and bonding the synthetic polymer film with a natural polymer film, the moldability and post-molding retention ability of the alveolar bone regeneration membrane manufactured, that is, the space retention ability can be implemented in a balanced manner, and the convenience of surgery by a practitioner can be maximized accordingly.

The effects of the present invention are not limited to the aforementioned effects and should be understood to include all possible effects deduced from the configuration of the invention described in the detailed description or the claims of the present invention.

### [Description of Drawings]

FIG. 1 illustrates a method for manufacturing an alveolar bone regeneration membrane according to an embodiment of the present invention.
FIG. 2 illustrates a method for preparing a natural polymer film according to an embodiment of the present invention.
FIG. 3 illustrates a method for laminating and bonding a synthetic polymer film and a natural polymer film according to an embodiment of the present invention.
FIGS. 4 and 5 are respective photographs of devices for measuring the space retention ability (bending strength) and moldability (elastic recovery rate) of the alveolar bone regeneration membranes according to Examples of the present invention and Comparative Examples.
FIG. 6 is a set of results of measuring the space retention ability (bending strength) and moldability (elastic recovery rate) of the alveolar bone regeneration membranes according to Examples of the present invention and Comparative Examples.
FIG. 7 is a photograph of a device for measuring the nutrient permeation ability of the alveolar bone regeneration membranes according to Examples of the present invention and Comparative Examples.
FIG. 8 is a set of results of measuring the nutrient permeation ability of the alveolar bone regeneration membranes according to Examples of the present invention and Comparative Examples.

### [Modes of the Invention]

Hereinafter, the present invention will be described with reference to the accompanying drawings. However, the present invention can be realized in various different forms, and is not limited to the embodiments described herein. In addition, in order to clearly describe the present invention, portions that are not related to the description are omitted in the drawings, and like reference numerals are added to like portions throughout the specification.

Throughout the specification, when one part is "connected" to another part, this includes not only a case where they are "directly connected to each other", but also a case where they are "indirectly connected to each other" with another member interposed therebetween. Further, when one part "includes" one constituent element, unless otherwise specifically described, this does not mean that another constituent element is excluded, but means that another constituent element may be further included.

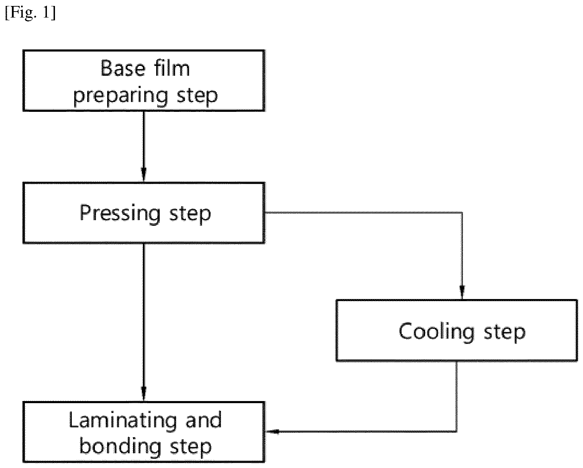

FIG. 1 illustrates a method for manufacturing an alveolar bone regeneration membrane according to an embodiment of the present invention. Referring to FIG. 1, the method for manufacturing an alveolar bone regeneration membrane according to an aspect of the present invention may include: (A) a step of preparing a base film by melting and molding a synthetic polymer resin; (B) a step of pressing the base film to prepare a first polymer film; and (C) a step of alternately laminating and bonding the first polymer film and a second polymer film containing a natural polymer resin; and may further include: a step of cooling the first polymer film between the steps (B) and (C), if necessary.

The average pore size of the base film and the first polymer film may be D₁ and D₂, respectively, and 0.1 ≤ D₂/D₁ ≤ 0.5.

The method for manufacturing an alveolar bone regeneration membrane may actually include: a step of preparing the first polymer film, a step of preparing the second polymer film, and a step of laminating and bonding the first and second polymer films.

### Preparation of first polymer film

The first polymer film may be prepared by (A) a step of preparing a base film by melting and molding a synthetic polymer resin and (B) a step of pressing the base film to prepare a first polymer film.

The synthetic polymer resin may be one selected from the group consisting of polylactic acid (PLA), polyglycolic acid (PGA), poly lactic-co-glycolic acid (PLGA), poly(L-lactic acid) (PLLA), polycaprolactone (PCL), poly(lactide-co-caprolactone) (PLCL), polyhydroxybutyrate (PHB), polyhydroxyvalerate (PHV), polydioxanone (PDO), poly(trimethylene carbonate) (PTMC), and combinations of two or more thereof, and may be preferably polycaprolactone (PCL), but is not limited thereto.

In the step (A), the molding may be performed by one selected from the group consisting of casting, melt compression, extrusion, injection, additive manufacturing, and combinations of two or more thereof, preferably additive manufacturing, that is, a 3D printing method. For example, the 3D printing may be performed using a fused deposition modeling (FDM) type 3D printer including one or more nozzles, and the nozzle may have a diameter of 0.1 to 0.5 mm. In this case, the nozzle may have a diameter of 0.1 mm or more, 0.2 mm or more, or 0.3 mm or more, and 0.5 mm or less. The diameter of the nozzle is a factor that directly or indirectly affects the pore characteristics of the first polymer film, specifically, the porosity and/or the pore size, and for example, the larger the diameter of the nozzle is, the larger the average pore size of the base film may be.

In the step (B), the pressurization may be performed at a pressure of 3 to 50 mPa. In this case, the pressure applied in the step (B) may be 3 mPa or more, or 30 mPa or more, and 50 mPa or less. The average pore size of the base film may be reduced by the pressurization, and with respect to the reducible range, for example, when the average pore size of the base film and the first polymer film is D₁ and D₂, respectively, 0.1 ≤ D₂/D₁ ≤ 0.5. In this case, the D₂/D₁ value may be 0.1 or more, 0.15 or more, or 0.2 or more, and 0.5 or less, 0.45 or less, or 0.4 or less. When the D₂/D₁ value is less than 0.1, the space retention ability of the membrane is good, but the elastic recovery rate deteriorates, so that moldability may become poor, and when the D₂/D₁ value exceeds 0.5, the moldability of the membrane is good, but the space retention ability may deteriorate. In other words, when the D₂/D₁ deviates from the above range, it is difficult to implement the moldability and space retention ability of the membrane in a balanced manner.

The method for preparing the first polymer film, for example, may include: a step of preparing a synthetic polymer resin raw material as a powder phase having an average particle size of 0.1 to 2 mm; preparing the synthetic polymer resin raw material into a base film having a thickness of 0.1 to 0.5 mm and an average pore size of 0.1 to 3 mm; and reducing the average pore size and thickness of the base film by cooling the base film for 3 minutes to 3 hours and pressing the base film at 3 to 50 mPa; but each step and condition are not limited thereto.

### Preparation of second polymer film

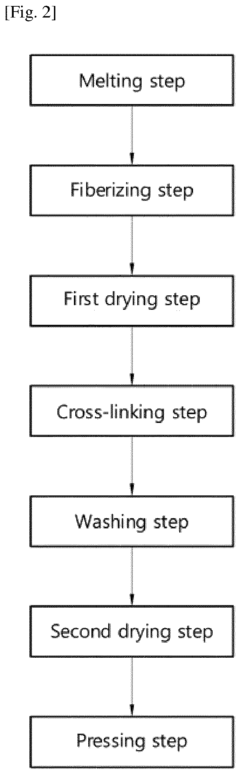

FIG. 2 illustrates a method for preparing a second polymer film according to an embodiment of the present invention. Referring to FIG. 2, the second polymer film may be prepared by a method including (b1) a step of dissolving a second polymer resin in an acid solution; (b2) a step of fiberizing the second polymer resin by introducing a salt into the product of the step (b1); (b3) a step of drying the product of the step (b2); (b4) a step of cross-linking the second polymer resin by introducing a cross-linking agent into the product of the step (b3); and (b5) a step of washing and drying the product of the step (b4) and then pressurizing.

In the step (b1), the acid solution may include one selected from the group consisting of phosphoric acid, sulfuric acid, hydrochloric acid, nitric acid, acetic acid, and combinations of two or more thereof, but is not limited thereto.

In the step (b2), the salt may be one selected from the group consisting of a phosphate, a calcium salt, a sodium salt, and combinations of two or more thereof, and may be preferably a phosphate in consideration of compatibility with the step (b1), but is not limited thereto.

In the step (b4), the cross-linking agent may be one selected from the group consisting of formaldehyde, a-hydroxyadipaldehyde, glutaraldehyde, 1-ethyl-3-(3-dimethylaminopropyl)-carbodiimide/N-hydroxy succinimide (EDC/NHS), and combinations of two or more thereof, and may be preferably glutaraldehyde, but is not limited thereto.

The natural polymer resin may be one selected from the group consisting of collagen, chitosan, hyaluronic acid, carboxymethyl cellulose, heparan sulfate, dextran, alginate, and combinations of two or more thereof, and may be preferably collagen, but is not limited thereto. The collagen may be extracted from one selected from the group consisting of porcine skin, porcine pericardium, porcine tendon, bovine skin, bovine pericardium, bovine tendon, and combinations of two or more thereof, but is not limited thereto.

In the step (b5), the washing may be performed using one selected from the group consisting of purified water, phosphate buffered saline (PBS), ethanol, and combinations of two or more thereof.

The method for preparing the second polymer film, for example, may include: a step of mixing a natural polymer resin and an acid solution at a concentration of 0.3 3 w/v% (w/v% = mg/ml% or kg/L%); fiberizing the mixed solution with a solution including a salt at 20 to 37°C for 12 to 48 hours; a step of obtaining a base film by lyophilizing a fiberized product for 24 to 72 hours; a step of chemically cross-linking the base film to a membrane for 6 to 48 hours using a cross-linking agent; washing the resulting film once to five times for 12 to 24 hours using a washing solution at 20 to 37°C after cross-linking; a step of drying the film by airdrying the film at 20 to 37°C for 3 to 24 hours; and a step of pressing the dried film at 20 to 80°C for 30 minutes to 12 hours, but each step and condition are not limited thereto.

### Lamination and bonding of first and second polymer films

In the step (C), an alveolar regeneration membrane, which is a final product may be prepared by alternately laminating and bonding the first polymer film and a second polymer film containing a natural polymer resin.

The step (C) may include: (c1) a step of alternately laminating the first and second polymer films; and (c2) a step of heating the product of the step (c1) to a temperature of 30 to 80°C and bonding the product at a pressure of 3 to 30 mPa. In this case, the heating temperature in the step (c2) may be 30°C or more, 40°C or more, or 50°C or more, and 80°C or less, 70°C or less, or 60°C or less. Further, the pressure applied in the step (c2) may be 3 mPa or more, 7 mPa or more, or 11 mPa or more, and 30 mPa or less, 26 mPa or less, or 22 mPa or less.

In addition, in the step (c1), the second polymer film including a natural polymer resin may be laminated and bonded on at least one surface, preferably both surfaces of the first polymer film.

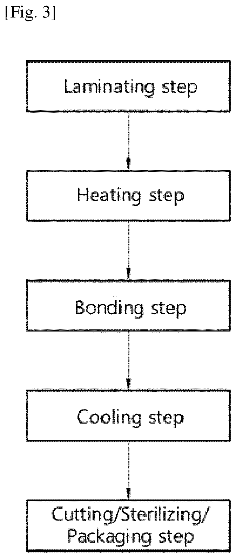

FIG. 3 illustrates a method for laminating and bonding a synthetic polymer film and a natural polymer film according to an embodiment of the present invention. Referring to FIG. 3, a method for laminating and bonding the first and second polymer films may include: for example, a step of alternately laminating the first and second polymer films into 2 to 10 layers and heating the films to 30 to 80°C; a step of bonding the first and second polymer films by pressing the first and second polymer films at a pressure of 3 to 30 mPa; a step of cooling a bonded membrane for 3 minutes to 3 hours; a step of cutting the cooled membrane into an appropriate size; and a step of sterilizing the cut membrane, and then packaging the sterilized membrane, but each step and condition are not limited thereto.

Hereinafter, the Examples of the present invention will be described in detail.

### Example 1

### Preparation of collagen film

20 g of collagen was dissolved in 1 L of an aqueous phosphoric acid solution, the collagen was fiberized by treating the resulting solution with a phosphate buffered saline (PBS) solution at 25°C for 20 hours, a sheet was prepared by lyophilizing the fiberized collagen for 40 hours, and the sheet was chemically crosslinked using a cross-linking agent glutaraldehyde. After the cross-linking, the sheet was washed to remove the cross-linking agent remaining on the sheet, and dried with warm air using wind at 25°C for 12 hours. A collagen film having a thickness of 0.2 mm was obtained by pressing the dried sheet at 50°C for 2 hours.

### Preparation of polymer film

Polycaprolactone (PCL) was supplied to a 3D printer nozzle having a diameter of 0.2 mm, the polycaprolactone was melted by heating the nozzle to 130°C, and then a base film having an average pore size of 1.2 mm was prepared by injecting the polycaprolactone at a pressure of 300 kPa. A polymer film having an average pore size of 0.5 mm was obtained by cooling the injected base film at 25°C for 5 minutes and then pressing the cooled base film at a pressure of 30 mPa.

### Manufacture of alveolar bone regeneration membrane

After the collagen film was laminated on both surfaces of the polymer film, an alveolar bone regeneration membrane having a three-layered structure of collagen/polycaprolactone/collagen (Col/PCL/Col) and an average pore size and a thickness of 0.2 mm and 0.4 mm, respectively, was manufactured by thermo-compressing the laminate under the conditions of 80°C and 20 mPa with a plate-type thermocompressor (Qmesys, QM900M) for 5 minutes.

### Example 2

An alveolar bone regeneration membrane was manufactured in the same manner as in Example 1, except that the diameter of the 3D printer nozzle was changed to 0.4 mm during the preparation of the polymer film.

### Example 3

An alveolar bone regeneration membrane was manufactured in the same manner as in Example 1, except that the average pore size of the alveolar bone regeneration membrane was adjusted to 0.4 mm by changing the pressure of the thermocompressor to 7 mPa during the manufacture of the alveolar bone regeneration membrane.

### Example 4

An alveolar bone regeneration membrane was manufactured in the same manner as in Example 1, except that the collagen film was laminated on one surface of the polymer film during the manufacture of the alveolar bone regeneration membrane.

### Example 5

An alveolar bone regeneration membrane was manufactured in the same manner as in Example 1, except that the temperature and pressure of the thermocompressor were changed to 50°C and 5 mPa, respectively, during the manufacture of the alveolar bone regeneration membrane.

### Example 6

An alveolar bone regeneration membrane was manufactured in the same manner as in Example 1, except that the temperature and pressure of the thermocompressor were changed to 50°C and 30 mPa, respectively, during the manufacture of the alveolar bone regeneration membrane.

### Example 7

An alveolar bone regeneration membrane was manufactured in the same manner as in Example 1, except that the pressure of the thermocompressor was changed to 5 mPa during the manufacture of the alveolar bone regeneration membrane.

### Example 8

An alveolar bone regeneration membrane was manufactured in the same manner as in Example 1, except that the pressure of the thermocompressor was changed to 30 mPa during the manufacture of the alveolar bone regeneration membrane.

### Comparative Example 1

An alveolar bone regeneration membrane (Bio-Gide™, manufactured by Geistlich, thickness 0.5 mm) made of a collagen material, which was commercially available, was prepared.

### Comparative Example 2

An alveolar bone regeneration membrane (OssGuide™, manufactured by SK Bioland Co., Ltd., thickness 0.5 mm) made of a collagen material, which was commercially available, was prepared.

### Comparative Example 3

An alveolar bone regeneration membrane was manufactured in the same manner as in Example 1, except that the preparation of the polymer film was changed as follows.

PLGA was melted at a temperature of 200°C before being moved into a barrel of an injection molding machine (Fanuc, i30A-injection molding machine), then placed in a molding portion in the form of a sheet and injection-molded at a pressure of 1,800 bar and a temperature of 200°C. A PLGA plate-type material was obtained by cooling the injection-molded PLGA at 25°C for 10 minutes, and then detaching the cooled PLGA from the molding portion.

### Comparative Example 4

An alveolar bone regeneration membrane was manufactured in the same manner as in Example 1, except that the base film was pressed at a pressure of 2 mPa during the preparation of the polymer film.

### Comparative Example 5

An alveolar bone regeneration membrane was manufactured in the same manner as in Example 1, except that the base film was pressed at a pressure of 60 mPa during the preparation of the polymer film.

The structural characteristics of each configuration and alveolar bone regeneration membrane manufactured according to the Examples and Comparative Examples are shown in the following Tables 1 to 3.

**[Table 1]**

| Classification | Example 1 | Example 2 | Example 3 | Example 4 |
|---|---|---|---|---|
| Laminated structure | Col/PCL/Col | Col/PCL/Col | Col/PCL/Col | Col/PCL |
| D₁ (mm) | 1.2 | 1.2 | 1.2 | 1.2 |
| PCL film pressurization pressure (mPa) | 30 | 30 | 30 | 30 |
| D₂ (mm) | 0.5 | 0.5 | 0.5 | 0.5 |
| D₂/D₁ | 0.42 | 0.42 | 0.42 | 0.42 |
| Membrane pressurization pressure (mPa) | 20 | 20 | 7 | 20 |
| Membrane pressurization temperature (°C) | 80 | 80 | 80 | 80 |
| Membrane average pore size (mm) | 0.2 | 0.2 | 0.4 | 0.2 |
| Membrane thickness (mm) | 0.4 | 0.4 | 0.4 | 0.4 |

**[Table 2]**

| Classification | Example 5 | Example 6 | Example 7 | Example 8 |
|---|---|---|---|---|
| Laminated structure | Col/PCL/Col | Col/PCL/Col | Col/PCL/Col | Col/PCL/Col |
| D₁ (mm) | 1.2 | 1.2 | 1.2 | 1.2 |
| PCL film pressurization pressure (mPa) | 30 | 30 | 30 | 30 |
| D₂ (mm) | 0.5 | 0.5 | 0.5 | 0.5 |
| D₂/D₁ | 0.42 | 0.42 | 0.42 | 0.42 |
| Membrane pressurization pressure (mPa) | 5 | 30 | 5 | 30 |
| Membrane pressurization temperature (°C) | 50 | 50 | 80 | 80 |
| Membrane average pore size (mm) | 0.4 | 0.3 | 0.25 | 0.15 |
| Membrane thickness (mm) | 0.4 | 0.4 | 0.4 | 0.4 |

**[Table 3]**

| Classification | Comparative Example 3 | Comparative Example 4 | Comparative Example 5 |
|---|---|---|---|
| Laminated structure | Col/PLGA | Col/PCL/Col | Col/PCL/Col |
| D₁ (mm) | 1.5 | 1.2 | 1.2 |
| PCL film pressurization pressure (mPa) | - | 2 | 60 |
| D₂ (mm) | 1.5 | 1.1 | 0.1 |
| D₂/D₁ | 1.00 | 0.92 | 0.08 |
| Membrane pressurization pressure (mPa) | 20 | 20 | 20 |
| Membrane pressurization temperature (°C) | 80 | 80 | 80 |
| Membrane average pore size (mm) | 0.2 | 0.2 | 0.2 |
| Membrane thickness (mm) | 0.4 | 0.4 | 0.4 |

### Experimental Example 1: Evaluation of space retention ability and moldability

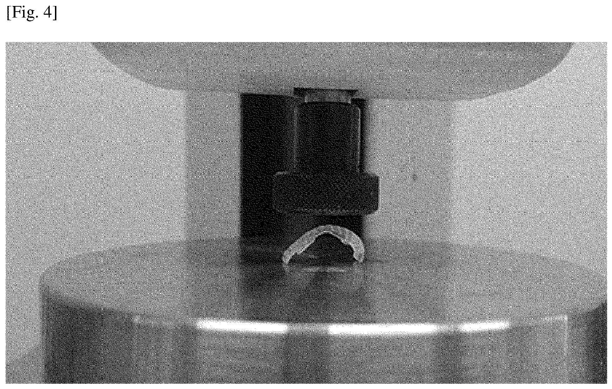

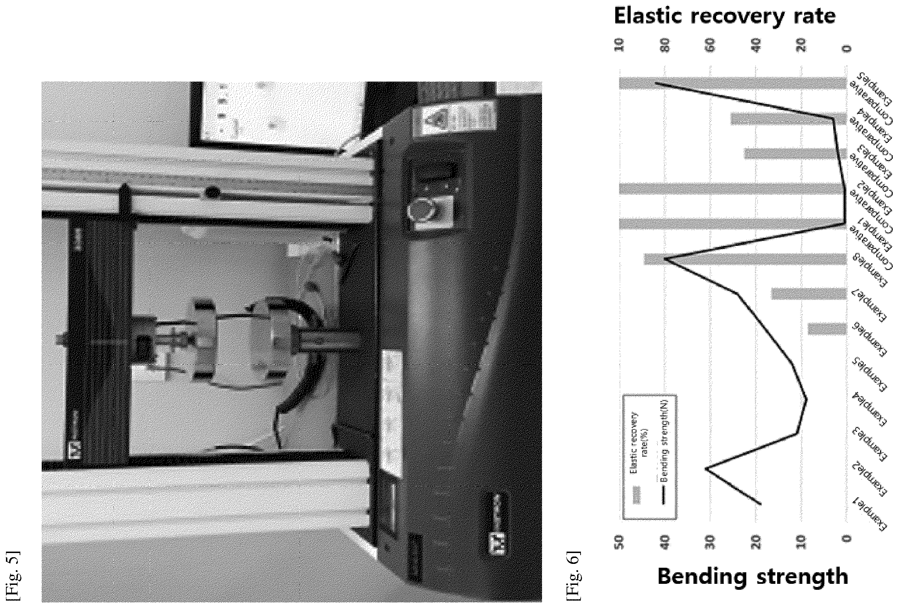

The space retention ability and moldability of the alveolar bone regeneration membranes according to the Examples and the Comparative Examples were compared and evaluated. The space retention ability was evaluated through bending strength using a push-pull gauge (HG-500) in FIG. 4, and the moldability was evaluated through an elastic recovery rate using a universal material tester (INSTRON 3365) in FIG. 5.

The following Table 4 and FIG. 6 shows the results of evaluating the space retention ability and moldability of the alveolar bone regeneration membranes according to the Examples and the Comparative Examples.

**[Table 4]**

| Classification | Bending strength (N) | Elastic recovery rate (%) |
|---|---|---|
| Example 1 | 19 | - |
| Example 2 | 31 | - |
| Example 3 | 11 | - |
| Example 4 | 9 | - |
| Example 5 | 12 | 0 |
| Example 6 | 18 | 17 |
| Example 7 | 24 | 33 |
| Example 8 | 40 | 89 |
| Comparative Example 1 | 0.3 | 100 |
| Comparative Example 2 | 0.5 | 100 |
| Comparative Example 3 | 2 | 45 |
| Comparative Example 4 | 3 | 51 |
| Comparative Example 5 | 42 | 100 |

Referring to FIG. 6, both Comparative Examples 1 and 2, which are existing alveolar bone regeneration membranes made of a collagen material, showed a bending strength of 1 N or less, but the bending strengths of the membranes according to Examples 1 to 4 were shown to be 9 N or more, so that an improved fixing force can be implemented compared to Comparative Examples 1 and 2.

Furthermore, it can be seen that Examples 5 to 8, in which the pore size of the membrane is adjusted within a predetermined range by adjusting the pressure and temperature during the bonding of the laminated membrane, has a bending strength of 12 N or more and simultaneously exhibits an elastic recovery rate of 90% or less, and thus has sufficient moldability at room temperature.

In contrast, it was found that Comparative Examples 3 to 5 failed to implement the space retention ability and moldability in a balanced manner. Specifically, the membranes of Comparative Examples 3 and 4 having a D₂/D₁ value of more than 0.5 have good moldability, but have remarkably poor space retention ability, and the membrane of Comparative Example 5 having a D₂/D₁ value of less than 0.1 has a good space retention ability, but shows an elastic recovery rate of 100%, and thus has remarkably poor moldability.

Through the results, it can be seen that the membranes of Examples 1 to 8, particularly, Examples 5 to 8 effectively and appropriately resolve the trade-off between space retention ability and moldability shown in the membranes of Comparative Examples 1 to 5, and thus can simultaneously implement them in a balanced manner.

### Experimental Example 2: Evaluation of membrane effectiveness

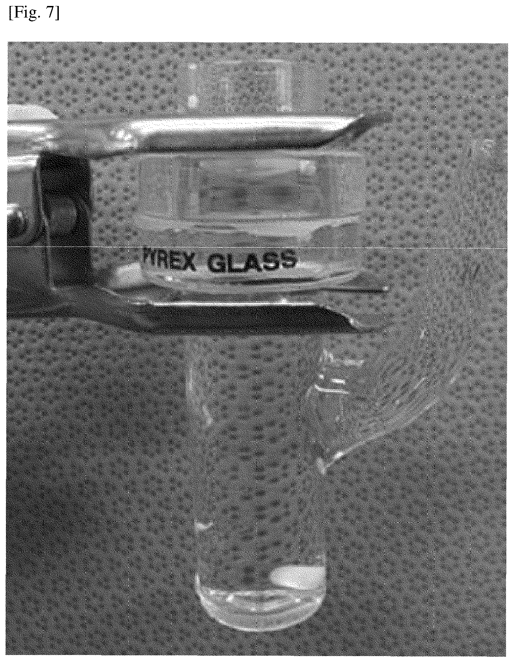

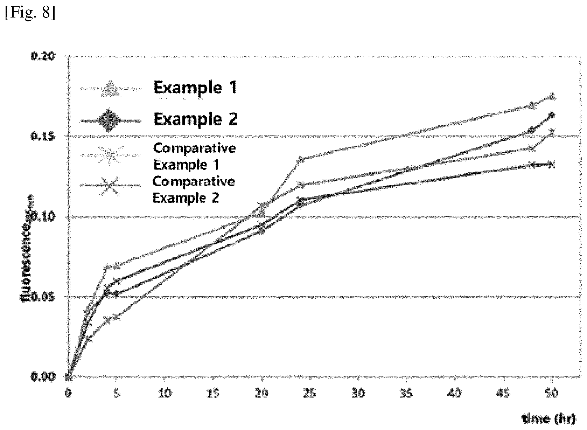

In order to confirm the nutrient permeation ability of the alveolar bone regeneration membranes according to the Examples and the Comparative Examples, an experiment was conducted using a diffusion cell as illustrated in FIG. 7. A BSA-FITC solution showing a strong wavelength at 485 nm was placed in the upper layer of a container, the degree to which the nutrient passed through the membrane and moved to the lower layer was analyzed using an absorbance measuring device, and the results are illustrated in FIG. 8.

Referring to FIG. 8, the membrane of Examples 1 and 2 have an ability to allow nutrients to pass through the membrane, which is equal to or more than those of Comparative Examples 1 and 2, which are existing alveolar bone regeneration membranes made of a collagen material, and particularly, the difference in permeation ability is more noticeably confirmed after about 40 hours have passed.

The above-described description of the present invention is provided for illustrative purposes, and a person skilled in the art to which the present invention pertains will understand that the present invention can be easily modified into other specific forms without changing the technical spirit or essential features of the present invention. Therefore, it should be understood that the above-described embodiments are only illustrative in all aspects and not restrictive. For example, each constituent element which is described as a singular form may be implemented in a distributed form, and similarly, constituent elements which are described as being distributed may be implemented in a combined form.

The scope of the present invention is represented by the claims to be described below, and it should be interpreted that the meaning and scope of the claims and all the changes or modified forms derived from the equivalent concepts thereto fall within the scope of the present invention.

## Claims

1. A method for manufacturing an alveolar bone regeneration membrane, the method comprising: (A) a step of preparing a base film by melting and molding a synthetic polymer resin; (B) a step of pressing the base film to prepare a first polymer film; and (C) a step of alternately laminating and bonding the first polymer film and a second polymer film containing a natural polymer resin, and satisfying the conditions below: the average pore size of the base film and the first polymer film are D₁ and D₂, respectively, and 0.1 ≤ D₂/D₁ ≤ 0.5.

2. The method of claim 1, wherein the synthetic polymer resin is one selected from the group consisting of polylactic acid (PLA), polyglycolic acid (PGA), poly lactic-co-glycolic acid (PLGA), poly(L-lactic acid) (PLLA), polycaprolactone (PCL), poly(lactide-co-caprolactone) (PLCL), polyhydroxybutyrate (PHB), polyhydroxyvalerate (PHV), polydioxanone (PDO), poly(trimethylene carbonate) (PTMC), and combinations of two or more thereof.

3. The method of claim 1, wherein in the step (A), the molding is performed by an additive manufacturing.

4. The method of claim 3, wherein the additive manufacturing is performed using a fused deposition modeling (FDM) type 3D printer comprising one or more nozzles.

5. The method of claim 4, wherein the nozzle has a diameter of 0.1 to 0.5 mm.

6. The method of claim 1, wherein in the step (B), the pressurization is performed at a pressure of 3 to 50 mPa.

7. The method of claim 1, wherein the natural polymer resin is one selected from the group consisting of collagen, chitosan, hyaluronic acid, carboxymethyl cellulose, heparan sulfate, dextran, alginate, and combinations of two or more thereof.

8. The method of claim 1, wherein the second polymer film is prepared by a method comprising the following steps: (b1) a step of dissolving a second polymer resin in an acid solution; (b2) a step of fiberizing the second polymer resin by introducing a salt into the product of the step (b1); (b3) a step of drying the product of the step (b2); (b4) a step of cross-linking the second polymer resin by introducing a cross-linking agent into the product of the step (b3); and (b5) a step of washing and drying the product of the step (b4) and then pressurizing.

9. The method of claim 8, wherein the acid solution comprises one selected from the group consisting of phosphoric acid, sulfuric acid, hydrochloric acid, nitric acid, acetic acid, and combinations of two or more thereof.

10. The method of claim 8, wherein the salt is one selected from the group consisting of a phosphate, a calcium salt, a sodium salt, and combinations of two or more thereof.

11. The method of claim 8, wherein the cross-linking agent is one selected from the group consisting of formaldehyde, a-hydroxyadipaldehyde, glutaraldehyde, 1-ethyl-3 -(3 -dimethylaminopropyl)-carbodiimide/N-hydroxy succinimide (EDC/NHS), and combinations of two or more thereof.

12. The method of claim 1, wherein the step (C) comprises the following steps: (c1) a step of alternately laminating the first and second polymer films; and (c2) a step of heating the product of the step (c1) to a temperature of 30 to 80°C and bonding the product at a pressure of 3 to 30 mPa.

13. The method of claim 1, wherein in the step (C), the second polymer film comprising the natural polymer resin is laminated and bonded on at least one surface of the first polymer film.
